(19) Europäisches Patentamt
European Patent Office
Office européen des brevets

(11) Publication number: **0 419 901 A1**

(12) # EUROPEAN PATENT APPLICATION

(21) Application number: **90117096.9**

(51) Int. Cl.⁵: **A61K 7/48**, A61K 7/42

(22) Date of filing: **05.09.90**

(30) Priority: **14.09.89 JP 239642/89**

(43) Date of publication of application:
**03.04.91 Bulletin 91/14**

(84) Designated Contracting States:
**DE FR GB IT**

(71) Applicant: **Sansho Seiyaku Co., Ltd.**
**26-7, Oike 2-chome**
**Onojo-shi Fukuoka-ken(JP)**

(72) Inventor: **Motono, Masahiro**
**272-5, Nishi-machi**
**Kurume-shi, Fukuoka-ken(JP)**

(74) Representative: **Patentanwälte Deufel- Schön-Hertel- Lewald**
**Isartorplatz 6**
**W-8000 München 2(DE)**

(54) **Endermic preparation for external application.**

(57) Disclosed is an endermic preparation for external application, which comprises a kojic acid or kojic acid derivatives as melanogenesis-inhibiting agents along with sodium hydrogensulfite and/or hydrogen peroxide as a discoloration-inhibiting agent. By addition of the discoloration-inhibiting agents, discoloration of kojic acid and its derivatives during storage thereof may effectively be prevented, and the high commercial value of the endermic preparation is maintained.

EP 0 419 901 A1

# ENDERMIC PREPARATION FOR EXTERNAL APPLICATION

## BACKGROUND OF THE INVENTION

### Field of the Invention

The present invention relates to an endermic preparation for external application, and more precisely, to that which contains kojic acid or kojic acid derivatives as active ingredients and which has been prevented from discoloration.

### Prior Art

As a result of years' research by the present inventors, it has been published that kojic acid or kojic acid derivatives are incorporated into various cosmetic materials and other endermic preparations for external application as active ingredients (JP-B-56-18569, 58-22151, 58-22152, 60-7961, 60-9722, 60-10005, 61-10447, 61-60801, 62-3820 and 63-24968 - the term "JP-B" as used herein means an "examined Japanese patent publication").

Since kojic acid and kojic acid derivatives have a function of inhibiting the action of tyrosinase existing in the human skin and exhibit an extreme melanin-inhibiting action, as so described in the above-mentioned Japanese patent publications, they are used as active ingredients in various endermic preparations for external application such as a skin-whitening cosmetic material. Accordingly, it is expected that they are to be further used as active ingredients in various endermic preparations for external application because of the useful properties thereof.

However, after kojic acid or kojic acid derivatives have been prepared into an endermic preparation for external application, the preparation has a problem that it would discolor to be pale yellow, though slightly, during the procedure of storage or distribution or in use or in keeping after partial use. Although the discoloration does not affect the melanin-inhibiting function of kojic acid and kojic acid derivatives at all, the problem of lowering the commercial value of the preparation is inevitable as the preparation is an endermic preparation for external application such as a cosmetic material.

Prior to the completion of the present invention, some means for preventing discoloration of kojic acid and kojic acid derivatives have been known.

For instance, JP-A-61-109705 (the term "JP-A" as used herein means an "unexamined published Japanese patent application") discloses a method for having kojic acid included in cyclodextrin so as to elevate the stability of kojic acid itself; and JP-A-63-270619 illustrates a means of incorporating kojic acid purified by sublimation into a preparation. However, it is impossible to incorporate a large amount of cyclodextrin into a preparation as it would cause a loss of the creamy texture of the preparation. On the other hand, only a slight amount of pure kojic acid can be obtained by sublimation, and there would be a little possibility of utilizing such sublimed kojic acid in a preparation for practical use and therefore the method of using the purified kojic acid could not always be desirable. JP-A-62-108804, 64-83008 and 63-188609 illustrate methods of incorporating various ultraviolet absorbents into preparations containing kojic acid or kojic acid derivatives. However, since the discoloration of kojic acid and kojic acid derivatives is caused not only by irradiation of ultraviolet rays but also by other factors such as heat, the methods could not be said to completely prevent discoloration of kojic acid-containing preparations.

## SUMMARY OF THE INVENTION

Accordingly, the object of the present invention is to provide an endermic preparation for external application which contains kojic acid or kojic acid derivative as an active ingredient and which is free from discoloring with the passage of time.

The present invention has been proposed for the purpose of attaining the above object and provides an endermic preparation for external application containing kojic acid or kojic acid derivatives, which is characterized by containing sodium hydrogensulfite and/or hydrogen peroxide as a discoloration-preventing

agent for kojic acid or kojic acid derivatives.

## DETAILED DESCRIPTION OF THE INVENTION

Both Sodium hydrogensulfite and hydrogen peroxide have a strong deoxidizing action and are known to of use as antioxidants or bleaching agents. It has been found that discoloration of kojic acid or kojic acid derivatives with the passage of time may noticeably be inhibited by incorporation of such sodium hydrogensulfite and/or hydrogen peroxide into kojic acid or kojic acid derivatives.

The proportion of sodium hydrogensulfite and/or hydrogen peroxide to be added to kojic acid or kojic acid derivatives is from 0.001 to 20.0 % by weight, preferably from 0.5 to 5.0 % by weight, to kojic acid or kojic acid derivatives. Where both sodium hydrogensulfite and hydrogen peroxide were used in combination,the ratio of sodium hydrogensulfite/hydrogen peroxide is from 0.001/0.0001 to 10.0/10.0, preferably from 0.0/0.1 to 5.0/5.0.

Kojic acid to be employed in the present invention includes a pure product of kojic acid, a fermented liquid containing kojic acid as a main component which is obtained by incubation of microorganisms having a kojic acid-producing ability, a concentrate obtained by concentrating said fermented liquid, and a crystallized kojic acid product obtained by extracting kojic acid from the fermented liquid and crystallizing the resulting extract. Particularly, when employing untreated fermented liquid, saccharides, peptide, amino acids and other unknown components which are in the liquid in a small amount could effectively act so as to further improve the melanin-inhibiting action of kojic acid and additionally to improve the stability of kojic acid against pH variation, heat and decoloration.

As examples of usable microorganisms having a kojic acid-producing ability, strains of genus Aspergillus, such as Aspergillus albus, Aspergillus candidus, Aspergillus oryzae, Aspergillus nidulans, Aspergillus parasiticus, Aspergillus awamori, Aspergillus tamarii, Aspergillus niveus, Aspergillus flavus, Aspergillus wentii, Aspergillus glaucus, Aspergillus clavatus, Aspergillus fumigatus, and Aspergillus giganteus; strains of the genus Penicillium such as Penicillium dalae; strains of the genus Escherichia such as Escherichia coli; strains of the genus Acetobacter such as Acetobacter xylinum; and strains of the genus Gluconobacter such as Gluconobacter roseus and Gluconobacter gluconicus are preferred. The composition of the medium to be used for incubating the microorganisms generally comprises from approximately 2 to 15 % (by weight - the same shall apply hereunder) of carbon sources such as saccharose, sucrose, D-fructose, glucose, starch, maltose, glycerin, mannitol, rhamnose, xylose, gluconic acid, arabinose, dihydroxyacetone, inositol, lactose and ethanol; from approximately 0.1 to 1 % of nitrogen sources such as ammonium sulfate, polypeptone, sodium nitrate, baker's yeast extract and beer yeast extract; from approximately 0.01 to 0.1 % of magnesium sources such as magnesium sulfate; and from approximately 0.001 to 0.005 % of inorganic salts such as ferric sulfate, ferric chloride, sodium chloride and calcium chloride.

Kojic acid derivatives to be used in the present invention include monoesters and diesters of kojic acid, as well as 2-benzoyloxymethyl-5-hydroxy-4H-pyran-4-one, 2-cinnamoyloxymethyl-5-hydroxy-4H-pyran-4-one, and 2-phenoxymethyl-5-hydroxy-4H-pyran-4-one.

As monoesters of kojic acid, the compounds which are illustrated in JP-B-58-22152 and represented by the formula:

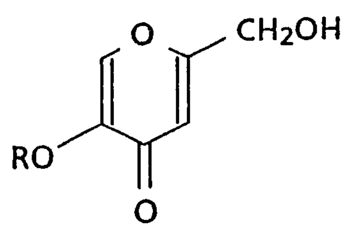

[where R represents an aliphatic acyl group having from 3 to 20 carbon atoms] are employed.

Such monoesters of kojic acid can be obtained, for example, by adding a chloride of an aliphatic carboxylic acid to a kojic acid-containing pyridine solution to cause esterification at room temperature, followed by isolation and purification of the resulting ester by chromatography or by extraction of the same with an organic solvent. Especially preferred monoesters of kojic acid are kojic acid monobutyrate, kojic acid monocaprylate, kojic acid monopalmitate and kojic acid monostearte.

As diesters of kojic acid, the compounds which are illustrated in JP-B-58-22151 and represented by the

formula:

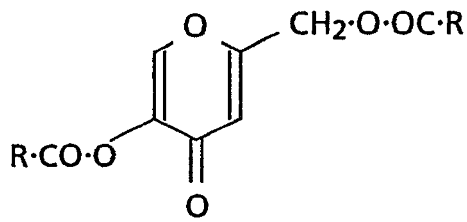

[where R represents a residue of an aliphatic carboxylic acid having from 3 to 20 carbon atoms wherein carboxyl group is removed] are employed.

Such diesters of kojic acids can be obtained easily, for example, by adding a chloride of an aliphatic carboxylic acid to a kojic acid-containing pyridine solution to cause esterification at room temperature. Especially preferred diesters of kojic acid are kojic acid dibutyrate, kojic acid dipalmitate, kojic acid distearate and kojic acid dioleate.

The endermic preparation for external application of the present invention may be in any form which is per se known, for example, an ointment, cream, an emulsion, dermatologic paste, cataplasm or a lotion. In any form, it is preferred that sodium hydrogensulfite and hydrogen peroxide are added to the preparation in the final stage of preparing the same, that is, after completion of the heating procedure.

For instance, in preparing an ointment preparation, an aqueous phase part is formed by adding hydrophilic components such as glycerin and sorbitol and kojic acid to a pure water, while separately from this, an oily phase part comprising solid oil components such as bee's wax, paraffin, microcrystalline wax, ceresine, higher fatty acids and hardened oil; semi-solid oil components such as vaseline, lanolin and glycerides; liquid oil components such as squalane, liquid paraffin and various ester oils; and an antiseptic and a surfactant is formed. Thus formed aqueous phase part is gently stirred and heated up to about 70 to 75 °C, and the oily phase part heated up to a temperature of the same degree is gradually added thereto. Then, resulting ointment is gradually cooled and sodium hydrogensulfite and/or hydrogen peroxide is added to the cooled ointment optionally along with a perfume.

As mentioned above, sodium hydrogensulfite and/or hydrogen peroxide is added to the preparation in the final stage of preparing the same, thereby they do not show deterioration due to heat and may display an excellent discoloration-inhibiting effect.

Examples of formulations of the endermic preparation for external application of the present invention are mentioned below.

| Formulation No. 1: Liquid Preparation | |
|---|---|
| Kojic acid | 0.50 (%) |
| Glycerin | 5.00 |
| Sorbitol | 4.00 |
| Stearic Acid Polyoxyl 40 | 1.55 |
| Ethanol | 10.00 |
| Sodium Hydrogensulfite | 0.05 |
| Pure Water | ~ 100 |

The above-mentioned ingredients were mixed, stirred and dissolved to obtain a liquid preparation.

| Formulation No. 2: Emulsion | |
|---|---|
| A. 1,3-Butylene Glycol | 4.00 (%) |
| Pure Water | ~ 100 |
| B. Soybean Lecithin | 0.80 |
| Ethanol | 5.00 |
| Polyethylene Glycol 400 | 6.00 |
| Pure Water | 20.00 |
| C. Pure Water | 1.00 |
| Hydrogen Peroxide (50 %) | 0.08 |
| Kojic Acid | 0.80 |

(A) and (B) were separately heated, and (A) was added to (B) and emulsified. During cooling the emulsified mixture, (C) was added thereto to obtain an emulsion.

| Formulation No. 3: Ointment | |
|---|---|
| A. Kojic Acid Monopalmitate | 1.00 (%) |
| Polysorbate 20 | 5.00 |
| Dextran | 0.07 |
| Ethanol | 5.00 |
| Hydrogen Peroxide (50 %) | 0.04 |
| B. White Petrolatum | ~ 100 |
| Beef Tallow | 20.00 |
| Cetanol | 2.00 |
| Glycerin Monostearate | 5.00 |

(A) was mixed and stirred. (B) was heated, mixed and dissolved. With cooling, (B) was mixed with (A) to prepare an ointment.

| Formulation No. 4: Milky Lotion | |
|---|---|
| A. White Petrolatum | 2.50 (%) |
| Silicone Oil | 0.50 |
| Stearyl Alcohol | 2.20 |
| Sucrose Fatty Acid Ester | 5.00 |
| Octyl Dodecanol | 2.00 |
| Squalane | 6.30 |
| Jojoba Oil | 0.20 |
| Stearic Acid Polyoxyl 40 | 2.50 |
| B. Pure Water | ~ 100 |
| Dipropylene Glycol | 3.00 |
| Alginic Acid Propylene Glycol | 0.30 |
| Xanthane Gum | 0.05 |
| C. Pure Water | 8.00 |
| Sodium Hydrogensulfite | 0.02 |
| Hydrogen Peroxide (50 %) | 0.04 |
| Kojic Acid Distearate | 1.00 |

(A) and (B) were separately heated and dissolved. (A) was added to (B) and emulsified. With cooling, (C) was added to the emulsified mixture to prepare a milky lotion.

| Formulation No. 5: Cream | |
|---|---|
| A. Pure Water | ~ 100 |
| 1,3-Butylene Glycol | 3.00 |
| Sorbitol | 7.00 |
| DL-PCA Sodium (50 %) | 3.00 |
| B. Polysorbate 60 | 2.50 |
| Monostearic Acid Glyceride | 1.50 |
| Cetanol | 3.00 |
| Vaseline | 5.00 |
| Octyldodecyl Myristate | 5.00 |
| Octyl Dodecanol | 6.00 |
| Squalane | 15.00 |
| C. Pure Water | 10.00 |
| Kojic Acid Moncbutyrate | 2.00 |
| Sodium Hydrogensulfite | 0.06 |
| Hydrogen Peroxide (50 %) | 0.04 |

(A) and (B) were separately heated and dissolved, and (B) was added to (A) and emulsified. With cooling, (C) was added to the emulsified mixture to prepare cream.

| Formulation No. 6: Pack Preparation | |
|---|---|
| A. Carboxyvinyl Polymer | 1.40 (%) |
| Dipropylene Glycol | 10.00 |
| Glycerin | 3.00 |
| Diisopropanolamine | 0.15 |
| B. Triacetin | 30.00 |
| Xanthane Gum | 2.00 |
| C. Pure Water | 3.00 |
| Sodium Hydrogensulfite | 0.03 |
| D. Pure Water | 10.00 |
| Kojic Acid | 1.50 |

(A) was mixed and dissolved to form a gel. Next, (B), (C) and (D) were added to (A) in order to prepare a pack preparation.

Next, the present invention will be explained in more detail by way of the following examples, which, however, are not intended to restrict the scope of the present invention.

| (A) Examples 1 to 6 | |
|---|---|
| Glycerin | 3.0 (%) |
| PG | 3.0 |
| Kojic Acid | 1.0 |
| Pure Water | ~ 100 (g) |
| Sodium Hydrogensulfite | 0.02 |
| (B) Examples 7 to 12 | |
| Glycerin | 3.0 (%) |
| PG | 3.0 |
| Polydoxyethylene Hardened Castor oil | 4.0 |
| Ethanol | 5.0 |
| Kojic Acid Derivative | 1.0 |
| Pure Water | ~ 100 (g) |
| Sodium Hydrogensulfite | 0.02 |

EXAMPLE 1:

As mentioned above, 0.02 % by weight of sodium hydrogensulfite was incorporated into an aqueous solution containing 1 % by weight of kojic acid. The resulting mixture was put in a glass container and set in a thermostat having a conditioned temperature of 50 °C and a conditioned humidity of 55 %. After 20 days, the absorbance of the mixture was measured and the degree of discoloration was observed.

The sample liquid was taken into a glass cell and the absorbance thereof at a wavelength of 420 nm was measured with a spectorphotometer (UVIDEC-200B, manufactured by Nippon Bunko Co.).

The result obtained is shown in Table 1 below.

EXAMPLES 2 TO 6:

In the formulation of Example 1, the proportion of sodium hydrogensulfite and/or hydrogen peroxide was varied, and the degree of discoloration of each mixture was evaluated in the same manner.

The results obtained are shown in Table 1 below.

COMPARATIVE EXAMPLES 1 TO 5:

The same formulations as those in Examples 1 to 6 were prepared, except that phytic acid or nicotinic acid amide was incorporated in place of sodium hydrogensulfite and/or hydrogen peroxide or none of them were incorporated. The degree of discoloration of each mixture was evaluated in the same manner.

The results obtained are shown in Table 1 below.

EXAMPLES 7 TO 12:

The same formulations as those in Example 1 were prepared, except that kojic acid monopalmitate (a kojic acid derivative) was incorporated in place of kojic acid. The degree of discoloration of each mixture was evaluated. The results obtained are shown in Table 2 below.

COMPARATIVE EXAMPLES 6 TO 10:

The same formulations as those in Examples 7 to 12 were prepared, except that phytic acid or nicotinic acid amide was incorporated in place of sodium hydrogensulfite and/or hydrogen peroxide, or none of them were incorporated.

The results obtained are shown in Table 2 below.

Table 1

| | Examples | | | | | | Comparative Examples | | | | |
|---|---|---|---|---|---|---|---|---|---|---|---|
| | 1 | 2 | 3 | 4 | 5 | 6 | 1 | 2 | 3 | 4 | 5 |
| Kojic Acid | 1.0 | 1.0 | 1.0 | 1.0 | 1.0 | 1.0 | 1.0 | 1.0 | 1.0 | 1.0 | 1.0 |
| Sodium Hydrogensulfite | 0.02 | 0.2 | | | | | | | | | |
| Hydrogen Peroxide (50 %) | | | 0.04 | 0.4 | | | | | | | |
| Sodium Hydrogensulfite plus Hydrogen Peroxide (50 %) (1/1) | | | | | 0.06 | 0.6 | | | | | |
| Phytic Acid (50 %) | | | | | | | 0.04 | 0.4 | | | |
| Nicotinic Acid Amide | | | | | | | | | 0.02 | 0.2 | |
| $OD_{420}$ | 0.112 | 0.037 | 0.057 | 0.011 | 0.024 | 0.010 | 0.365 | 0.652 | 0.212 | 0.399 | 0.255 |

Table 2

| | Examples | | | | | | Comparative Examples | | | | |
|---|---|---|---|---|---|---|---|---|---|---|---|
| | 7 | 8 | 9 | 10 | 11 | 12 | 6 | 7 | 8 | 9 | 10 |
| Kojic Acid Derivative | 1.0 | 1.0 | 1.0 | 1.0 | 1.0 | 1.0 | 1.0 | 1.0 | 1.0 | 1.0 | 1.0 |
| Sodium Hydrogensulfite | 0.02 | 0.2 | | | | | | | | | |
| Hydrogen Peroxide (50 %) | | | 0.04 | 0.4 | | | | | | | |
| Sodium Hydrogensulfite plus Hydrogen Peroxide (50 %) (1/1) | | | | | 0.06 | 0.6 | | | | | |
| Phytic Acid (50 %) | | | | | | | 0.04 | 0.4 | | | |
| Nicotinic Acid Amide | | | | | | | | | 0.02 | 0.2 | |
| $OD_{420}$ | 0.087 | 0.043 | 0.063 | 0.026 | 0.033 | 0.011 | 0.315 | 0.475 | 0.195 | 0.345 | 0.201 |

In accordance with the present invention, discoloration of an endermic preparation for external application which contains kojic acid or kojic acid derivatives as active ingredients having a melanogenesis-inhibiting activity can be prevented during storage or after storage for a long period of time, by incorporation of sodium hydrogensulfite and/or hydrogen peroxide as a discoloration-inhibitor. Accordingly, the commercial value of the endermic preparation of the present invention is not lowered during or after storage thereof. The present invention is therefore extremely useful in preparing an endermic preparation for external application containing a kojic acid or kojic acid derivatives.

While the invention has been described in detail and with reference to specific embodiments thereof, it will be apparent to one skilled in the art that various changes and modifications can be made therein without departing from the spirit and scope thereof.

## Claims

1. An endermic preparation for external application, which comprises a kojic acid or kojic acid derivatives

along with sodium hydrogensulfite and/or hydrogen peroxide.

2. The endermic preparation for external application as in claim 1, in which the proportion of sodium hydrogen sulfite and/or hydrogen peroxide is from 0.001 to 20.0 % by weight to kojic acid or a kojic acid derivatives.

3. The endermic preparation for external application as in claim 2, in which the proportion of sodium hydrogensulfite and/or hydrogen peroxide is from 0.5 to 5.0 % by weight to kojic acid or a kojic acid derivative.

4. The endermic preparation for external application as in claim 1, in which the kojic acid derivative is one selected from the group consisting of monoesters and diesters of kojic acid, 2-benzoyloxymethyl-5-hydroxy-4H-pyran-4-one, 2-cinnamoyloxymethyl-5-hydroxy-4H-pyran-4-one and 2-phenoxymethyl-5-hydroxy-4H-pyran-4-one.

5. The endermic preparation for external application as in claim 4, in which monoesters of kojic acid represented by the formula:

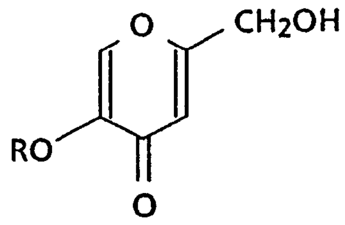

where R represents an aliphatic acyl group having from 3 to 20 carbon atoms.

6. The endermic preparation for external application as in claim 4, in which diesters of kojic acid represented by the formula:

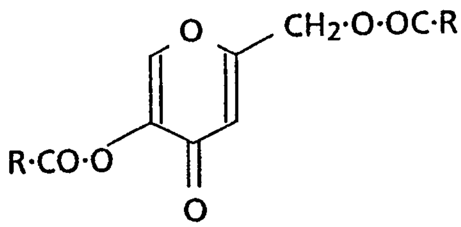

where R represents a residue of an aliphatic carboxylic acid having from 3 to 20 carbon atoms wherein carboxyl group is removed.

7. The endermic preparation for external application as in any one of claims 1 to 6, which is in the form of an ointment, cream, an emulsion, dermatologic paste, cataplasm or a lotion.

8. The endermic preparation for external application as in any one of claims 1 to 6, which is prepared by adding sodium hydrogensulfite and/or hydrogen peroxide in the final stage of preparing the preparation.

9. The endermic preparation for external application as in claim 8, which is prepared by adding sodium hydrogensulfite and/or hydrogen peroxide after completion of the heating in preparing the preparation.

European
Patent Office

# EUROPEAN SEARCH REPORT

Application Number

**EP 90 11 7096**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 300 842 (MOET-HENNESSY RECHERCHE)<br>* Example 10; claims *<br>- - - | 1-3,7 | A 61 K 7/48<br>A 61 K 7/42 |
| Y | | 1-3,5-9 | |
| Y | US-A-4 692 261 (FILOMENO)<br>* Claims; example 2; column 5, lines 19-39 *<br>- - - | 1-3,5-9 | |
| Y,D | GB-A-2 052 973 (SANSHO PHARMACEUTICAL)<br>* Whole docment<br>* & JP-A-61 60 801<br>- - - | 1-3,5-9 | |
| A | CHEMICAL ABSTRACTS, vol. 103, no. 26, December 1985, page 343, abstract no. 220623r, Columbus, Ohio, US;<br>& JP-A-60 146 821 (SANSEI PHARMACEUTICAL CO., LTD) 02-08-1985<br>* Abstract *<br>- - - | 1-9 | |
| A | CHEMICAL ABSTRACTS, vol. 100, no. 26, June 1984, page 329, abstract no. 215312x, Columbus, Ohio, US;<br>& JP-A-59 33 207 (SANSEI PHARMACEUTICAL CO., LTD) 23-02-1984<br>* Abstract *<br>- - - - - | 1,4 | **TECHNICAL FIELDS SEARCHED (Int. Cl.5)**<br>A 61 K |

The present search report has been drawn up for all claims

| Place of search | Date of completion of search | Examiner |
|---|---|---|
| The Hague | 18 January 91 | FISCHER J.P. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document